# EUROPEAN PATENT APPLICATION

(11) **EP 3 722 410 A1**
(43) Date of publication of application: **14.10.2020**
(21) Application number: 20165482.9
(22) Date of filing: 25.03.2020
(51) Int. Cl.: C12M 1/24, C12M 1/04, C12M 1/34, C12M 1/36

(54) **AUTOMATED SYSTEMS AND METHODS FOR CELL CULTURING**

(30) Priority: 29.03.2019 US 201962826279 P
(71) Applicant: Fenwal, Inc., Lake Zurich, IL 60047 (US)
(72) Inventor: WEGENER, Christopher J., Lake Zurich, IL Illinois 60047 (US); SCHLINKER, Alaina, Lake Zurich, IL Illinois 60047 (US)
(74) Representative: Maikowski & Ninnemann Patentanwälte Partnerschaft mbB

(57) **Abstract**

Systems and methods for automated cell culturing are disclosed. The system includes a stand-alone device including a culturing vessel and an integrated controller for delivering fluids at pre-determined times and sensors for monitoring the conditions of the culturing environment. The sensors for monitoring the conditions are coupled to the controller.

## Description

### Field of the Disclosure

The present disclosure is directed to the culturing of biological cells. More particularly, the present disclosure is directed to automated systems and methods for the culturing of biological cells. Even more particularly, the present disclosure is directed to methods and systems for monitoring and controlling the conditions of a biological cell culture in a stand-alone culturing system.

### Background

The culturing of cells is common in the field of biotechnology. Cell culturing refers to the growing of biological cells in a controlled environment. In cell culturing, cells of interest that have been isolated from living tissue are placed in a controlled environment for a period of time where they are maintained and grown for later use in, for example, a therapy. The cells are placed in a vessel such as a flask or a Petri dish and maintained in a suitable environment which typically includes a liquid, gel or other medium that provides the cells with necessary nutrients for cell growth.

In addition to providing the cells with a nutrient-containing medium, the cells must also reside in a suitable gas and temperature environment. Gases such as CO₂ and O₂ must be carefully supplied (or removed) to allow the cells to grow. Furthermore, factors such as pH, media volume must also be monitored and regulated to ensure suitable cell growth.

Traditionally, the culturing of cells has been carried out in a suitable vessel, such as the previously mentioned flask or dish, and kept in a temperature, humidity and CO₂ controlled incubator for a required period of time. As the cells grow, they tend to "outgrow" these traditional and small vessels and must often be "split" when the cells achieve a pre-determined cell concentration of culture saturation. "Splitting" of cells often requires that medium be moved and added and will, in general, increase handling frequency, labor and the possible risk of contamination and/or cell loss.

More recently, cell culturing vessels such as those described in U.S. Patent 9,255,243 have been developed which reduce the need to split cells. Vessels such as those described in U.S. Patent 9,255,243allow for a sufficient volume of culture medium to be added to the vessel over the course of the culturing process, thereby avoiding the need for cell splitting and some of the accompanying manual and handling systems that may otherwise be required. Vessels of the type shown and described in U.S. Patent 9,255,243 include a gas-permeable membrane at the bottom end of the vessel through which gases such as CO₂ and O₂ enter and exit the vessel chamber.

While vessels such those described in U.S. Patent 9,255,243 have simplified the culturing process to a degree, the culturing must still take place in a traditional humidity-, temperature-, and CO₂-controlled incubator. Moreover, most operations associated with the culturing of cells such as loading the vessel with cells, media, culture additives (cytokines, supplements, antibiotics, etc.), culture sampling, cell monitoring and cell harvesting must be performed manually. For example, the addition of culture reagents to the culture vessel may be triggered only following the drawing and analysis of a manually collected sample.

Thus, it would be desirable to provide a system and method of cell culturing that does not need to be performed in its entirety or at all, in a traditional humidity-, temperature-, and CO2-controlled incubator. In other words, it would be desirable to provide a "stand-alone" cell culturing system. The term "stand-alone" generally refers to a system or method that is independent of traditional incubation wherein the vessel need not reside, and the cell culturing need not be carried out in a traditional incubator.

It would also be desirable to provide a more automated system and method of biological cell culturing wherein certain operations such as the delivery or loading of the vessel with cells, media, culturing additives need not be manually performed but can instead be pre-programmed into the system's control system to be carried out at a preselected time. It would also be desirable to provide systems and methods wherein the conditions within the culture vessel can be monitored and controlled to provide a suitable culturing environment for a given cell population. Based on such monitoring, conditions may be automatically adjusted or corrected, as needed. For example, it would be desirable to monitor the cell concentration/number, temperature, pressure, and/or gas content in the vessel and adjust such conditions accordingly.

The systems and methods described herein address these needs.

### Summary

There several aspects to the subject matter described herein.

In one aspect, a system for the culturing of biological cells is described. The system includes a culturing vessel having a top cap and a bottom support member defining a culture chamber wherein the bottom support member includes a gas permeable membrane. The system further includes a base assembly configured to receive the bottom support member in a gas-tight, mating manner. The base assembly further includes one or more conduits for delivering to and/or removing one or more gases from the base. The system includes a culture medium source and a control system that has one or more sensors for sensing at least one of temperature or pressure during cell culturing.

In another aspect, an automated method for culturing biological cells is disclosed. The method includes monitoring certain conditions within a culturing vessel having a gas-permeable membrane at the base of said vessel and automatically adjusting the conditions based on said monitoring.

In a more particular aspect, a method for automatically controlling the conditions of a biological cell culturing environment is disclosed. The method includes placing one or more sensors at the interface of a culturing vessel and base docking unit for receiving the vessel and communicating selected conditions detected by said sensors to a controller coupled to said base docking unit. The method includes adjusting, as necessary, one or more conditions of said culturing environment based on the detected selected conditions.

### Brief Description of the Drawings

Figure 1 is a schematic view of a cell culture system as described herein;
Figure 2 is a schematic view of the cell culture vessel of Fig.1 with the lower collar shown in cross-section; and
Figure 3 is a diagram of a control system for the system described herein.

### Detailed Description of the Embodiments

The embodiments disclosed herein are for the purpose of providing a description of the present subject matter, and it is understood that the subject matter may be embodied in various other forms and combinations not shown in detail.

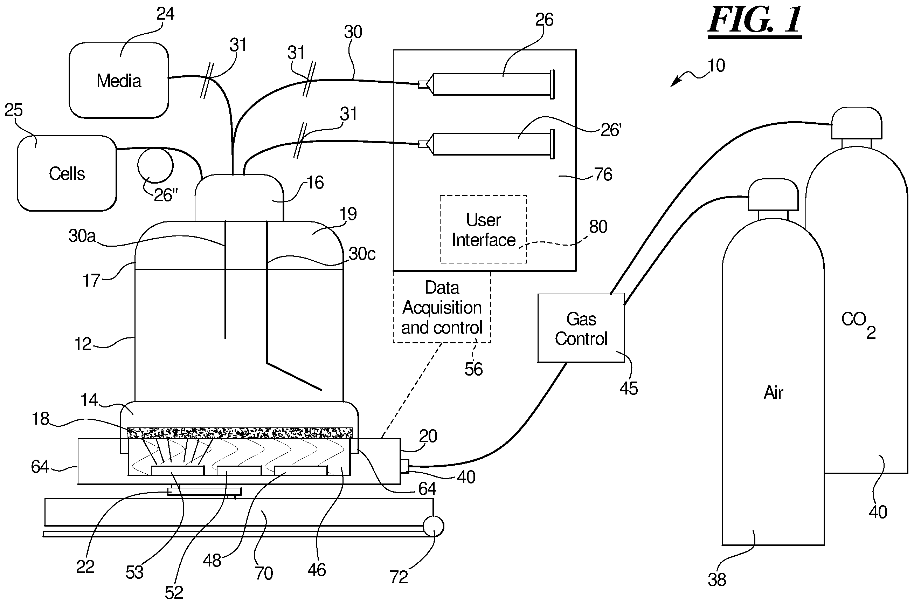

Figure 1 is a schematic view of system 10 described herein. As discussed above, system 10 may be a "stand-alone" system that does not require a traditional incubator for maintaining and controlling the culturing of the biological cells. Alternatively, the system may allow for certain steps in the overall cell culturing process to be carried out in a traditional incubator while allowing other steps to be performed outside of the incubator under the direction of the integrated controller of system 10. Thus, for example, system 10, as described in greater detail below, may be automated and include an integrated controller for monitoring the culturing conditions and delivering and removing fluid at predetermined times and/or in response to certain conditions.

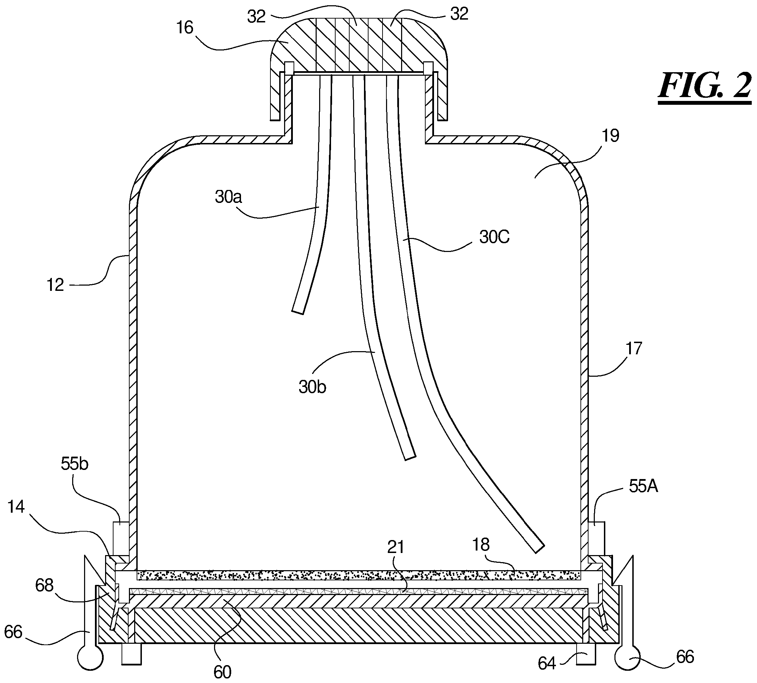

As shown in Figure 1, system 10 includes a culture vessel 12. Culture vessel 12 includes a lower collar 14 and a top cap 16. Collar 14 includes or carries a gas-permeable membrane 18 and a mesh support 21, as shown in Figure 2. Vessel 12 is preferably cylindrical and includes wall 17, preferably transparent, which together with collar 14 (and membrane 18) and cap 16 define the chamber 19 of vessel 12. Vessel 12 preferably is made of a plastic or polymeric material and is preferably disposable. Examples of specific dimensions, volumes and materials of vessel 12 may be found in, for example, in U.S. Patent No. 9,255,243.

Collar 14 of vessel 12 may be configured to allow for direct attachment to base docking unit 20 shown in Figure 1. Attachment of collar 14 (and thus vessel 12) may be by any means that provide in air-tight seal between collar 14 and base docking unit 20. By air-tight, it is meant that gas cannot escape from base docking unit 20 to the outside environment. (Of course, the transfer of gases between base docking unit 20 and chamber 19 of vessel 12 through membrane 18 is intentional and desired.) In one embodiment, mating of vessel 12 to base docking unit 20 may be through a locking arrangement as generally shown in Figure 2. For example, vessel 12 may include a rigid support member 60 at the bottommost end of vessel 12. Rigid support member 60 includes a circumferential, downwardly extending tongue that mates with a circumferential sealing member 64 in base docking unit 20. A clip or other retainer 66 may be used to secure vessel 12 to base docking unit 20. As shown in Fig. 2, clip 66 catches shoulder 68 in collar 14 while the other end of clip 66 is held by base docking unit 20. Other means for securing vessel 12 to base docking unit 20 in an air-tight manner may also be used.

Base docking unit 20 may be portable and suitable for direct placement on a flat surface. As shown in Figure 1, base docking unit 20 may be attached to and mechanically coupled to agitation assembly 22. In one embodiment, as shown in Fig. 1, base docking unit 20 may be mounted to a cam (or series of cams) and motor(s) in docking unit 20. The motor(s), once activated would cause docking base unit 20 to move in a linear or circular motion, thereby gently mixing the contents of vessel 12. In addition, system 10 may also include a pivot 72 that allows tilting (manual or automated) of base docking unit 20 and, more specifically, vessel 12 to aid in the harvesting of cells. Tilting of vessel 12 directs cells to flow toward a corner of the vessel where the "deepest" tube 30c has its terminal open end. This allows for more complete drawing of fluid out of vessel 12 during the harvesting of cells. Accordingly, system 10 may include a pivotable plate 70 to which base docking unit 20 is attached. Plate 70 may be tilted (typically less than 45° and preferably up to about 30°) about pivot 72.

As further shown in Figure 1, system 10 will include media source 24 and may further include additional containers 25 of fluids used during the culturing process such as the cells themselves or culture additives. System 10 may further include one or more pumps 26 for delivering the culture media, cells or other fluids to vessel 12. Fig. 1 shows several variants of "pumps" that may be used, either separately or in combination in the system disclosed herein. For example, pumps 26 may be syringes which draw fluid from media source 24 or, alternatively may be prefilled as shown by syringe 26' with the appropriate media source or other liquid. In another alternative example, pumps 26" may be traditional peristaltic pumps or other pumps including compressible diaphragms or plates. Whether the pumping units are syringes, peristaltic pumps or other types of pumps, they may be mounted on a device panel 76 or console as shown schematically in Figure 1.

As noted above, while Fig. 1 shows syringes 26 or 26', mounted on panel 76, it will be understood that other types of pumps described herein such as peristaltic pumps 26" may likewise be mounted on console/panel 76. In addition, containers 24 and 25 may be suspended from hooks or hangers on console 76 or from a separate IV pole. Where containers 24 and 25 are suspended from hooks/hangers on console 76, such hooks may be coupled to weight scales that can monitor changes in weight in the containers. As further shown in Figure 1, pumps and media sources 24 and containers 25 of other liquids communicate with the chamber 19 of vessel 12 by flexible tubing 30 which define flow paths from the containers or pumps (syringes) and extend through channels 32 in cap 16 of vessel 12. Tubings 30a-30c define flow paths for delivering and withdrawing liquids to and from the chamber of vessel 12. Tubings 30 may be of varying length, with tubing 30c extending most deeply into chamber and is used for harvesting cells with or without tilting of vessel 12, as described above. In an alternative, vessel 12 may include tubing segments that extend out of cap 16 and into chamber 19 of vessel 12. These tubing segments may be joined, in a known sterile fashion, to tubing segments associated with the containers/syringes of media and other liquids. The sterile connections or sterile docks are schematically shown and identified by reference numerals 31.

As further shown in Figure 1, system 10 may include sources of CO₂ and O₂ gas for delivery to chamber 19 of vessel 12 through gas-permeable membrane 18. Gas sources 38 and 40 are fluidly connected to base docking unit 20 by gas lines 42 through ports 44 in unit 20. The flow of gas may be controlled manually by gas control 45 or otherwise preprogrammed into controller 56 for automatic control.

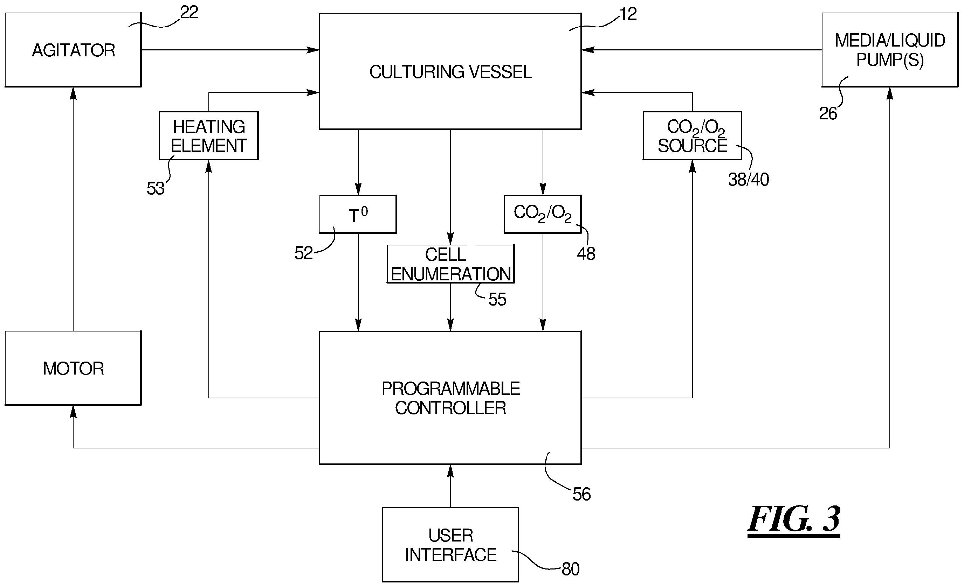

System 10 may further include one or more sensors for monitoring the environment within culturing vessel 12. As shown schematically in Figure 1, the interface area 46 between gas permeable membrane 18 and base docking unit 20 may house one or more sensors as shown in Figure 1. Preferably, sensors are placed as close to chamber 19 to most accurately detect conditions within chamber 19. For example, system 10 may be equipped with a pressure sensor 48 which measures the pressure at the gas permeable membrane 18 and/or the composition of the gas delivered to vessel 12. A temperature sensor 52 may also be included for determining or monitoring the temperature within chamber 19 of vessel 12. Temperature sensor 52 may be configured to measure the air temperature in the space between docking unit 20 and vessel 12. Alternatively, temperature sensor 52 may provide for non-contact measurement (such as by non-contact infrared sensors) to determine the temperature of vessel 12. If necessary, vessel 12 may further be equipped with heating element 51 (Fig. 3) which, in response to an inadequate temperature reading detected by sensor 52 may be activated and deliver additional heat to heat the gases or the inner surface of vessel 12.

System 10 may further include a sensor for sensing the number of cells in chamber 19 of vessel and cell growth generally. Such a "cell enumeration" sensor 53 may be a sensor whereby data is obtained in a non-invasive manner so that disturbance of the cells or cell culture may be avoided. For example, in one embodiment, system 10 may include an optical detector that measures light reflectance and light scattering properties of the cells and culture medium within chamber 19 to determine cell load. As shown in Fig. 1, cell enumeration sensor 53 may be a reflection-based sensor and emitter located in base docking unit 20. In this example, base 14 of vessel 12 may be provided with a lens or other element aligned with sensor 53 to allow for transmission of light through the bottom of vessel 20.

In another embodiment, base docking unit 20 may house (near the side of vessel 12) a light emitting diode 55a of a known wavelength as well as linear or two-dimensional sensor 55b or a CCD, as generally shown in Fig. 2. Whereas cell enumeration sensor 53, described above, may more directly determine the number of cells (e.g., cell density) in vessel 12, the "side" array of sensors 55a (alone or in combination with 55b) generally shown in Fig. 2 may be used to determine culture media components (such as glucose) or cell culture by-products (such as lactose) which may be correlated to a cell number. In a further alternative embodiment, sensor 55a may also be a reflection-based, combined emitter and receiver as described above in connection with cell enumeration sensor 53. Other non-invasive means may include ultrasonic scanning of the cell culture or measuring the capacitive properties of the cell culture.

In an alternative means for determining cell growth, vessel 12 may be equipped with a sensor for measuring pH or dissolved oxygen in the culture. In another embodiment of cell enumeration sensing, the system may be programmed to remove a sample of the culture media through one of tubes 30a-30c by activation of pump 26 (syringe). The sample may be analyzed by system 10 or analyzed offline to determine the state of cell growth through either direct cell count or indirect means (glucose/lactate concentration). The result may be entered into or electronically transferred to the system (controller 56) wherein the system may automatically (or by the operator) take appropriate action. In any event, whether non-invasive or invasive means are used, the sensed cell enumeration may provide feedback to controller 56 which may trigger reagent addition, agitation, gas delivery, temperature adjustment or other system-controlled elements.

The functions of system 10 are in large part controlled by system controller 56 in conjunction with a user interface 80 as shown in Figure 3. Controller 56 and user interface 80 may be housed in console 76 or base docking unit 20. While controller 56 may take the form of one or more electrical components or circuits, controller 56 comprises a processor and an associated memory according to one embodiment. According to such an embodiment, the processor may be programmed to carry out any of the actions that controller 56 is described as being configured to perform below. The instructions by which the processor is programmed may be stored on the memory associated with the processor, which memory may include one or more tangible computer readable memories, having computer executable instructions stored thereon, which when executed by the processor, may cause the one or more processors to carry out one or more actions.

As further shown In Fig. 3, controller may be coupled to one more sensors 46, 52 and/or 55 as described above. Information or data regarding temperature, pressure and cell enumeration is conveyed from the sensors to the controller 56. In response to the data provided by the sensors, controller may, as necessary, adjust the rate of delivery of CO₂ and/or O₂ gas to chamber 19 of vessel 12 from sources 38 and/or 40. As noted above, if the temperature detected by heat sensor 52 is inadequate, controller 56 may be pre-programmed to activate heating element 53. Controller 56 may also be programmed to activate the motor of agitation assembly 22 or activate pumps 26 to deliver additional reagent (medium) to vessel 12 in response to detected "cell enumeration" readings.

Thus, in accordance with the present disclosure, system may be pre-programmed to deliver cells, media and supplements to vessel 12; control the conditions of the cell culture including pH, temperature, % CO₂ and monitor cell growth (enumeration) without significant operator invention. Alternatively, system 10 may be used in a more temporary fashion in conjunction with more traditional cell culturing systems. In this example, vessel 12 could be removed from a conventional incubator at a pre-set time, docked to base docking unit 20 so that above-described sensors may be activated to take measurements, add or exchange media using the fluidic controls of system 10 based on the sensed readings/measurements. Vessel 12 may then be "undocked" from system 10 and returned to the incubator for further cell culturing and ultimate harvesting.

### OTHER EXAMPLES

Aspects of the present subject matter described above may be beneficial alone or in combination with one or more other Aspects, as described below.
Aspect 1. A system for the culturing of biological cells including: a culturing vessel having a top cap and a bottom support member defining a culturing chamber, the bottom support member carrying a gas permeable membrane. The system further includes a base assembly configured to receive the bottom support member in a gas-tight, mating manner, the base assembly further including one or more channels for delivering to and/or removing from the base one or more gases, and/or a culture medium source. The system includes a control system with one or more sensors for sensing at least one of temperature or pressure during cell culturing.
Aspect 2. The system of Aspect 1 further including one or more tubes joined to the cap and extending into the culturing chamber, the one or more tubes defining a flowpath for the culture medium.
Aspect 3. The system of any one of Aspects 1 or 2 wherein the base assembly further includes an agitator.
Aspect 4. The system of Aspect 3 wherein the agitator includes a platform for tilting the vessel.
Aspect 5. The system of any one Aspects 1 through 4 wherein the control system includes a pressure sensor for monitoring gas pressure at the interface of the gas permeable membrane and the base assembly.
Aspect 6. The system of any one of Aspects 1 through 5 wherein the base assembly further includes a heating element.
Aspect 7. The system of any one of Aspects 1 through 6 wherein the system further comprises a temperature sensor for measuring the temperature in the system.
Aspect 8. The system of Aspect 7 wherein the temperature sensor is located in the base member.
Aspect 9. The system of Aspect 8 wherein the controller is configured to adjust the temperature in the system in response to a reading from said temperature sensor.
Aspect 10. The system of any one of Aspects 1 through 9 further including a sensor for measuring the content of one of or more gases in the system.
Aspect 11. The system of Aspect 10 wherein the sensor for measuring the content of one of one or more gases is located in the base.
Aspect 12. The system of Aspect 11 wherein the controller is configured to adjust the amount of one or more gases in response to a reading of the sensor for measuring the content of the one or more gases.
Aspect 13. The system of any one of Aspects 1 through 12 further including sources of CO₂ and O₂ gas.
Aspect 14. The system of Aspect 13 wherein the sources of CO₂ and O₂ gas are delivered to the base assembly by fluid lines joined to inlets in the base assembly.
Aspect 15. The system of Aspect 14 wherein the controller is configured to control the flow of gas to the base assembly.
Aspect 16. The system of any one of Aspects 1 through 15 further including one or more pumping devices for delivering and withdrawing culture media and/or biological cells to the culturing vessel.
Aspect 17. The system of Aspect 16 wherein the controller is configured to deliver or withdraw one or both of the culture media and/or biological cells.
Aspect 18. The system of Aspect 3 wherein the controller is configured to actuate the agitator during culturing.
Aspect 19. The system of any one of Aspects 1 through 18 including a cell enumeration sensor.
Aspect 20. An automated method for culturing biological cells including monitoring certain conditions within a culturing vessel having a gas-permeable membrane at the base of said vessel. The method includes automatically adjusting the conditions based on the monitoring.
Aspect 21. A method for automatically controlling the conditions of a biological cell culturing environment including sensing the conditions of a biological cell culturing with one or more sensors at or near the interface of a culturing vessel and base unit for receiving said vessel; communicating selected conditions detected by said sensors to a controller coupled to said base unit; and adjusting, as necessary, one or more conditions of the culturing environment based on the detected selected conditions.
Aspect 22. The method of Aspect 21 including sensing the temperature at the interface of the culturing vessel and the base unit.
Aspect 23. The method of Aspect 22 including adjusting the temperature at the interface.
Aspect 24. The method of Aspect 21 including sensing the temperature in the culturing vessel.
Aspect 25. The method of Aspect 21 including measuring the number of cells and/or cell growth in the vessel.
Aspect 26. The method of Aspect 25 including measuring the number of cells and/or cell growth in the vessel by removing a sample of culture media from the vessel.
Aspect 27. The method of Aspect 21 including adjusting the delivery of gas to the culturing vessel.
Aspect 28. The method of Aspect 21 further including replacing a culture medium in the vessel.
Aspect 29. The method of Aspect 21 further including agitating the culturing vessel.
Aspect 30. The method of Aspect 29 including agitating the culturing vessel by tilting the vessel.

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter of the invention.

## Claims

1. A system for the culturing of biological cells comprising:
(a) a culturing vessel comprising a top cap and a bottom support member defining a culturing chamber, said bottom support member carrying a gas permeable membrane;
(b) a base assembly configured to receive said bottom support member in a gas-tight, mating manner, said base assembly further comprising one or more channels for delivering to and/or removing from said base one or more gases;
(c) a culture medium source; and
(d) a control system comprising one or more sensors for sensing at least one of temperature or pressure during cell culturing.

2. The system of Claim 1 further comprising one or more tubes joined to said cap and extending into said culturing chamber, said one or more tubes defining a flow path for said culture medium.

3. The system of any one of Claims 1 or 2 wherein said base assembly further comprises an agitator.

4. The system of any one Claims 1 through 3 wherein said control system comprises a pressure sensor for monitoring gas pressure at the interface of said gas permeable membrane and said base assembly.

5. The system of any one of claims 1 through 4 wherein said base assembly further comprises a heating element.

6. The system of any one of Claims 1 through 5 wherein said system further comprises a temperature sensor for measuring the temperature in said system and said controller is configured to adjust the temperature in said system in response to a reading from said temperature sensor.

7. The system of any one of Claims 1 through 6 further comprising a sensor for
measuring the content of one of or more gases in said system and said controller is configured to adjust the amount of said one or more gases in response to a reading of said sensor for measuring the content of said one or more gases.

8. The system of any one of Claims 1 through 7 further comprising one or more pumping devices for delivering and withdrawing culture media and/or biological cells to said culturing vessel.

9. The system of Claim 8 wherein said controller is configured to deliver or withdraw one or both of said culture media and/or biological cells.

10. The system of any one of Claims 1 through 9 comprising a cell enumeration sensor.

11. A method for automatically controlling the conditions of a biological cell culturing environment comprising:
a) sensing the conditions of a biological cell culturing with one or more sensors at or near the interface of a culturing vessel and base unit for receiving said vessel;
b) communicating selected conditions detected by said sensors to a controller coupled to said base unit; and
c) adjusting, as necessary, one or more conditions of said culturing environment based on said detected selected conditions.

12. The method of Claim 11 comprising sensing the temperature said culturing vessel.

13. The method of Claim 11 comprising measuring the number of cells and/or cell growth in said vessel.

14. The method of Claim 11 comprising adjusting the delivery of gas to said culturing vessel.

15. The method of Claim 11 further comprising replacing a culture medium in said vessel.
